# FASCICULE DE BREVET EUROPEEN

(11) **EP 2 039 326 B1**
(45) Date de publication et mention de la délivrance du brevet: **29.04.2015**
(21) Numéro de dépôt: 08164669.7
(22) Date de dépôt: 19.09.2008
(51) Int. Cl.: A61F 2/30

(54) **Composant de prothèse articulaire et prothèse articulaire comprenant un tel composant**
Komponente einer Gelenkprothese und eine solche Komponente umfassende Gelenkprothese
Artificial joint component and artificial joint comprising such a component

(30) Priorité: 19.09.2007 FR 0757675
(43) Date de publication de la demande: 25.03.2009
(73) Titulaire: Tornier, 38330 Montbonnot-Saint-Martin (FR)
(72) Inventeur: Rollet, Vincent, 38420, SAINT JEAN LE VIEUX (FR)
(74) Mandataire: Myon, Gérard Jean-Pierre

(56) Documents cités:
- EP-A- 0 679 375
- EP-A- 1 088 532
- EP-A- 1 679 050
- WO-A-2007/039820
- US-A- 2 203 219
- US-A- 4 093 011

## Description

La présente invention a trait un composant de prothèse articulaire ainsi qu'à une prothèse articulaire comprenant un tel composant. L'invention a également trait à une méthode de pose d'un tel composant.

Dans le domaine des prothèses articulaires, notamment des prothèses d'épaules, il est connu de constituer un composant huméral en assemblant une queue d'ancrage et une partie métaphysaire au moyen d'une vis de fixation. Dans certain cas, une entretoise est prévue entre la queue d'ancrage et la partie métaphysaire, de façon à donner au composant la longueur souhaitée en fonction de l'anatomie du patient.

Dans la mesure où une prothèse est installée pour une longue période, il est important de garantir la rigidité et la stabilité mécanique d'un composant de prothèse. Or, dans les systèmes connus, il est possible que, du fait des sollicitations répétées sur le composant, la vis de fixation ait tendance à se desserrer, ce qui peut conduire à une instabilité, voire à un désassemblage du composant, ce phénomène pouvant être accentué par des vibrations. Dans les matériels connus, l'immobilisation de la partie métaphysaire par rapport à la queue d'ancrage résulte principalement du serrage de la vis. Si la vis n'est pas assez serrée lors de la pose de la prothèse, le risque de désassemblage du composant prothétique est accentué.

EP-A-1 679 050 mentionne le principe de blocage d'une vis de commande du déplacement d'une bague en forme de coin qui dilate une partie élargie d'une queue d'ancrage de prothèse. Cette disposition est spécifique du blocage par bague en forme de coin. Les moyens permettant d'obtenir un tel blocage ne sont pas décrits.

C'est à ces inconvénients qu'entend plus particulièrement remédier l'invention en proposant un nouveau composant de prothèse articulaire dans lequel un dévissage non souhaité de la vis de fixation peut être évité.

A cet effet, l'invention concerne un composant de prothèse articulaire comprenant une queue d'ancrage apte à être ancrée dans un os, une partie métaphysaire destinée, à supporter un élément formant une surface articulaire de la prothèse ou formant elle-même surface articulaire, une vis de fixation de la partie métaphysaire sur la queue d'ancrage, ainsi que des moyens de blocage en rotation de la vis par rapport à la partie métaphysaire. Ce composant est **caractérisé en ce que** les moyens de blocage en rotation comprennent une pièce de blocage apte à venir en engagement, d'une part, avec au moins un relief correspondant d'une tête de la vis et, d'autre part, avec au moins un relief correspondant de la partie métaphysaire.

Grâce à l'invention, la pièce de blocage évite que la vis se desserre de façon intempestive, ce qui garantit la stabilité mécanique du composant. La pièce de blocage est efficace même si la vis de fixation n'est pas vissée à fond dans le taraudage correspondant.

Selon des aspects avantageux mais non obligatoires de l'invention, un tel composant peut incorporer une ou plusieurs des caractéristiques suivantes :
- La pièce de blocage est pourvue d'au moins un organe en saillie apte à être introduit entre une tête de la vis et la partie métaphysaire, au voisinage d'un logement de réception de cette tête en configuration assemblée du composant.
- La pièce de blocage comprend au moins deux organes en saillie aptes à être introduits simultanément entre la tête de la vis et la partie métaphysaire, de part et d'autre de cette tête.
- La surface radiale externe de la tête de la vis est pourvue de reliefs aptes à coopérer avec le ou chaque organe en saillie pour solidariser en rotation la tête de la vis et la pièce de blocage. Ces reliefs sont avantageusement des cannelures concaves, de forme complémentaire de celle du ou des organe(s) en saillie, le ou chaque organe en saillie étant partiellement engagé dans l'une des cannelures en configuration assemblée du composant.
- La partie métaphysaire comprend une ou des encoches en nombre égal au nombre d'organes en saillie, alors que la ou chaque encoche débouche dans le logement de réception de la tête de vis et que la ou chaque encoche est apte à recevoir partiellement le ou l'un des organes en saillie, en configuration assemblée du composant.
- Il est prévu des moyens de solidarisation de la pièce de blocage et de la vis dans une configuration où les moyens de blocage empêchent la rotation de la vis autour de son axe longitudinal. Ces moyens de solidarisation peuvent comprendre un taraudage ménagé dans la tête de la vis, ainsi qu'une vis auxiliaire apte à être vissée dans ce taraudage en plaquant la pièce de blocage contre la tête de la vis, dans une configuration où le ou les organe(s) en saillie sont disposés entre la tête de la vis et la partie métaphysaire.
- La pièce de blocage est pourvue, d'une part, d'au moins un premier relief apte à coopérer avec au moins un relief correspondant de la tête de la vis pour solidariser en rotation la pièce de blocage et la vis et, d'autre part, d'au moins un second relief apte à coopérer avec au moins un relief fixe par rapport à la partie métaphysaire pour bloquer la rotation de cette pièce de blocage.
- Selon certains modes de réalisation, la pièce de blocage est une bague, alors que son premier relief est interne et que le relief correspondant est un relief radial externe de la tête de la vis. Dans ce cas, le premier relief de la bague peut être une dent, alors que le relief correspondant de la tête de la vis est une encoche. En variante, la bague est équipée d'une denture périphérique interne, qui forme plusieurs premiers reliefs, alors que la tête de la vis est équipée d'une denture périphérique externe qui forme plusieurs reliefs correspondant aux premiers reliefs de la bague.
- Selon d'autres modes de réalisation, la pièce de blocage est un bouchon qui recouvre la tête de la vis, alors que le premier relief est ménagé sur un côté du bouchon tourné vers la tête de la vis et que le relief correspondant est ménagé sur la face de la tête de la vis opposée à sa tige. La tête de la vis peut porter au moins une projection en saillie par rapport à sa face opposée à la tige, alors que le bouchon est équipé d'au moins un logement de réception de cette projection. En variante, le bouchon est équipé d'une denture qui forme plusieurs premiers reliefs, alors que la face de la tête de la vis opposée à sa tige est équipée d'une denture qui forme plusieurs reliefs correspondant au premier relief du bouchon.
- Le deuxième relief de la pièce de blocage s'étend sur le, ou à partir, du bord périphérique externe de cette pièce.
- Une entretoise peut être disposée entre la queue d'ancrage et la partie métaphysaire, cette entretoise étant pourvue, sur ses faces opposées tournées respectivement vers la queue d'ancrage et vers la partie métaphysaire, de reliefs destinés à venir en prise avec des reliefs correspondants ménagés respectivement sur la queue d'ancrage et sur la partie métaphysaire, en empêchant une rotation de la partie métaphysaire par rapport à la queue d'ancrage, autour d'un axe longitudinal de l'entretoise.

L'invention concerne également une prothèse articulaire, en particulier une prothèse d'épaule, qui comprend un composant tel que mentionné ci-dessus. Une telle prothèse est plus fiable et plus facile à installer pour un chirurgien que celles de l'état de la technique.

L'invention sera mieux comprise et d'autres avantages et caractéristiques de celle-ci apparaîtront plus clairement à la lumière de la description qui va suivre donnée uniquement à titre d'exemple et faite en référence aux dessins annexés dans lesquels :
- La figure 1 est une représentation schématique en perspective partiellement éclatée d'un composant huméral de prothèse d'épaule conforme à l'invention ;
- La figure 2 est une vue de face du composant de la figure 1 en configuration assemblée ;
- La figure 3 est une coupe selon la ligne III-III à la figure 2 ;
- La figure 4 est une vue à plus grande échelle du détail IV à la figure 3 ;
- La figure 5 est une coupe selon la ligne V-V à la figure 3 ;
- La figure 6 est une vue à plus grande échelle du détail VI à la figure 5.
- La figure 7 est une coupe partielle à plus grande échelle selon la ligne VII-VII à la figure 6 ;
- La figure 8 est une vue en perspective des moyens de blocage du composant des figures 1 à 7 en cours de montage ;
- la figure 9 est une vue partielle en perspective de certains éléments d'un composant conforme à un deuxième mode de réalisation de l'invention ;
- la figure 10 est une vue de dessus des éléments représentés à la figure 9 en configuration assemblée du composant ;
- la figure 11 est une vue en perspective de certains éléments d'un composant conforme à un troisième mode de réalisation de l'invention ;
- la figure 12 est une vue en perspective de certains éléments d'un composant conforme à un quatrième mode de réalisation de l'invention ; et
- la figure 13 est une vue en perspective de certains éléments d'un composant conforme à un cinquième mode de réalisation de l'invention.

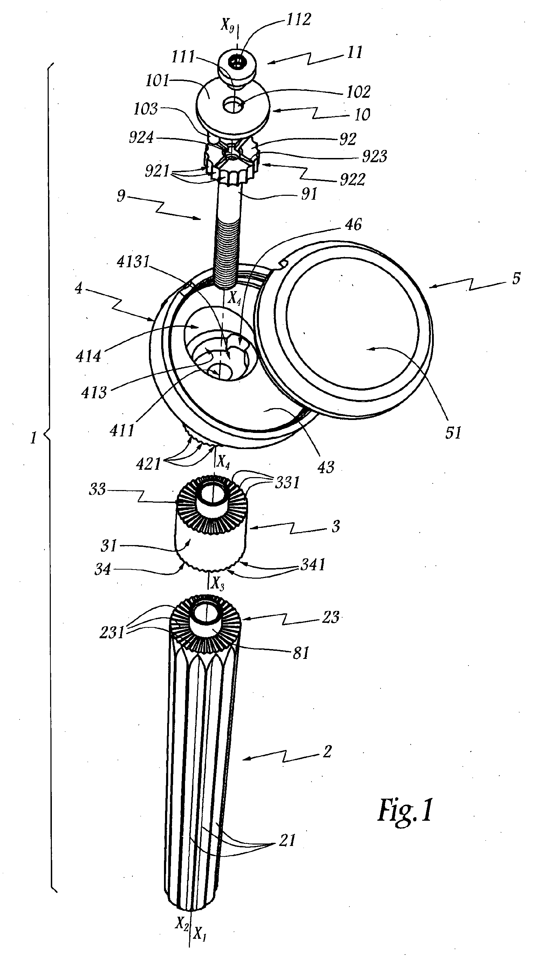

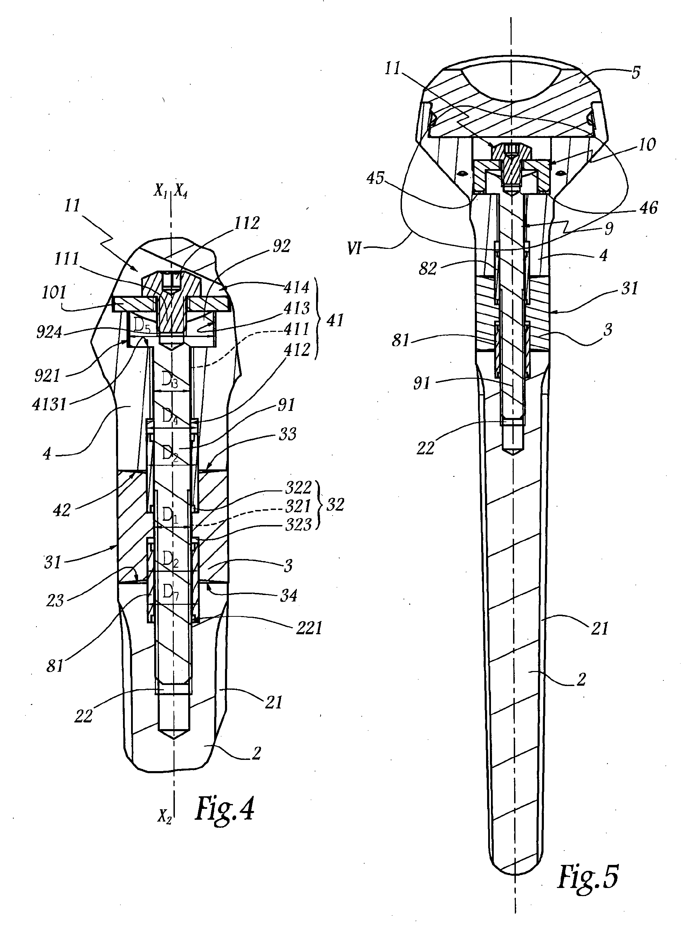

Le composant huméral 1 de prothèse d'épaule représentée aux figures 1 à 6 comprend une queue d'ancrage 2 destinée à être introduite et ancrée dans le canal médullaire d'un humérus, après résection de son extrémité supérieure. La queue 2 est pourvue de cannelures externes 21 destinées à faciliter son immobilisation en rotation à l'intérieur du canal médullaire. La queue 2 est pourvue d'un taraudage axial 22 qui s'étend le long de son axe longitudinal X₂.

Le composant 1 comprend également une entretoise 3 destinée à permettre l'ajustement en longueur du composant 1 en tenant compte de la morphologie du patient et de la profondeur d'enfoncement de la queue 2 dans le canal médullaire huméral. L'entretoise 3 peut être choisie dans un jeu d'entretoises de hauteurs différentes et de diamètres différentes.

L'entretoise 3 a une paroi radiale externe 31 cylindrique à base circulaire. L'entretoise 3 a également un perçage central 32 dont l'axe longitudinal X₃ est destiné à être aligné sur l'axe X₂ en configuration montée du composant 1. L'axe X₃ est également l'axe de symétrie de la surface 31. Le perçage 32 comprend une portion médiane 321 présentant un premier diamètre D₁ ainsi que deux portions 322 et 323 formant respectivement le débouché du perçage 32 sur la face supérieure 33 et la face inférieure 34 de l'entretoise 3. Les portions 322 et 323 ont un même diamètre D₂ qui est supérieur au diamètre D₁. Ainsi, la portion médiane 321 du perçage 32 forme deux épaulements au fond des portions débouchantes 322 et 323.

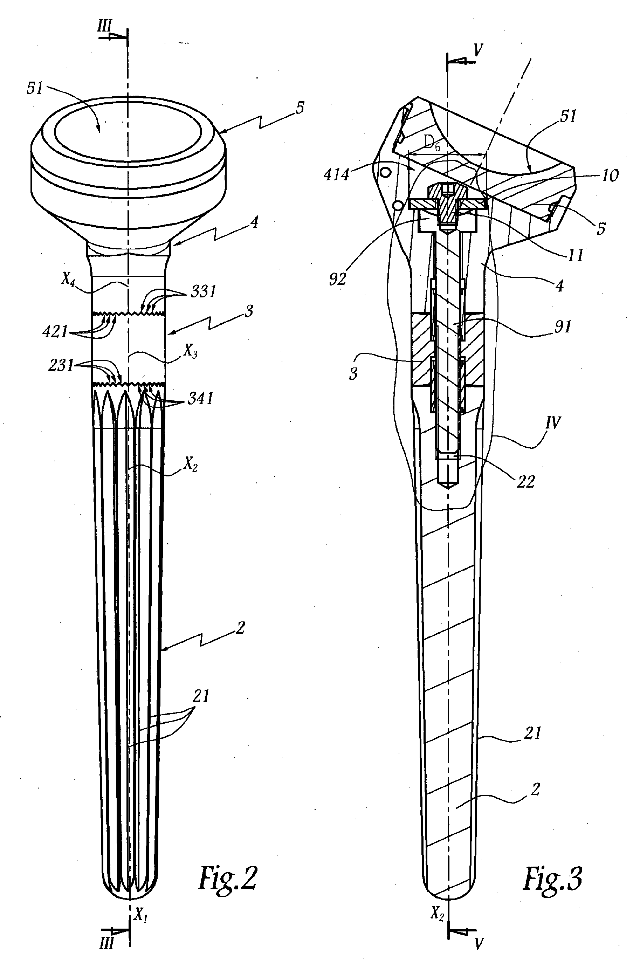

La face 33 de l'entretoise 3 est pourvue de rainures 331 qui s'étendent radialement autour de l'axe X₃. La face 34 est pourvue de rainures 341 du même type dont seule la trace est visible aux figures 1 et 2.

La face supérieure 23 de la queue 2 sur laquelle débouche le taraudage 22 est pourvue de rainures radiales 231 qui coopèrent avec les rainures 341 pour immobiliser en rotation, autour des axes X₂ et X₃ confondus, l'entretoise 3 par rapport à la queue 2 lorsque les surfaces 34 et 23 sont en appui l'une sur l'autre.

Le composant 1 comprend également une partie métaphysaire 4 qui forme un support pour un insert 5 définissant une surface articulaire concave 51 destinée à interagir avec une surface articulaire convexe qui appartient à un composant glénoïdien de la prothèse d'épaule ou à un composant intermédiaire, lorsqu'il est fait usage d'un tel composant intermédiaire.

La partie métaphysaire 4 est pourvue d'un perçage longitudinal 41 centré sur un axe X₄ destiné à être aligné avec les axes X₂ et X₃ en configuration montée du composant 1. Le perçage 41 comprend une portion médiane 411 dont on note D₃ le diamètre. Le perçage 41 comprend également une portion 412 débouchante sur une face 42 destinée à être en appui sur la face 33 de l'entretoise 3 en configuration montée du composant 1. On note D₄ le diamètre de la portion 412. Ce diamètre D₄ est sensiblement égal au diamètre D₂. Le perçage 41 comprend une troisième portion 413 qui jouxte la portion 411 et s'étend à l'opposé de la portion 412 par rapport à cette portion 411. On note D₅ le diamètre de cette portion 413. Le perçage 41 comprend enfin une quatrième portion 414 qui débouche sur une face 43 de la partie 4 qui est inclinée par rapport à l'axe X₄ et qui définit le fond du logement de réception de l'insert 5 dans la partie 4. On note D₆ le diamètre de la portion 414. Le diamètre D₅ est supérieur au diamètre D₄ et inférieur au diamètre D₆.

Le taraudage 22 ne s'étend pas jusqu'au niveau de la face 23 de la queue 2 dans la mesure où un lamage 221 est réalisé dans la zone de jonction entre le taraudage 22 et la face 23. Le lamage 221 a un diamètre D₇ sensiblement égal au diamètre D₂. En fait, les portions 322, 323, 412, 413 et 414 des perçages 32 et 41 sont également des lamages réalisés respectivement autour des portions médianes 321 et 411.

Des rainures 421 s'étendent radialement sur la face 42 de la partie 4, autour de l'axe X₄. Ces rainures ont une forme analogue à celle des rainures 331 avec lesquelles elles peuvent venir en prise.

Lors de l'assemblage du composant 1, deux bagues 81 et 82 sont respectivement serties dans les lamages 221 et 322. Les bagues 81 et 82 permettent de réaliser une immobilisation transversale relative des parties 2, 3 et 4 du composant 1 en cours de montage. En pratique, les bagues 81 et 82 sont identiques.

Lorsque ces parties 2, 3 et 4 sont en appui les unes sur les autres, il est possible de faire tourner la partie 4 autour de l'axe X₄, en écartant les surfaces 23 et 34 ou 33 et 42 l'une de l'autre, afin de régler l'orientation angulaire de l'insert 5 par rapport aux axes X₁, X₂ et X₃ qui définissent alors l'axe longitudinal X₁ du composant 1.

Lorsque cet assemble temporaire est réalisé, une vis 9 est introduite successivement dans le perçage 41 puis dans le perçage 31 et dans le taraudage 22. On note 91 la tige de la vis 9 et 92 sa tête. On note X₉ l'axe longitudinal de la tige 91, lequel est confondu avec les axes X₁, X₂, X₃ et X₄ en configuration monté du composant 1.

La tête 92 est pourvue de cannelures périphériques 921 ménagées sur sa face radiale externe 922. La tête 92 est également pourvue de quatre rainures 923 lui permettant de coopérer avec un tournevis plat ou cruciforme pour son entraînement en rotation autour de l'axe X₉ lors de son vissage dans le taraudage 22. Lors de l'assemblage du composant 1, la vis 9 est vissée dans le taraudage 22 jusqu'à ce que sa tête 92 vienne fermement en appui contre le fond 4131 de la portion 413 du perçage 41. Les éléments constitutifs du composant 1 sont alors fermement maintenus en position les uns par rapport aux autres, les rainures 231 et 341, d'une part, 331 et 341, d'autre part, étant en prise les unes avec les autres, ce qui contribue à l'immobilisation en rotation relative des parties 2, 3 et 4 autour des axes X₁, X₂, X₃ et X₄ confondus.

Pour éviter un desserrage accidentel de la vis 9, sous l'effet des sollicitations répétées du composant 1, et notamment des vibrations qu'il peut subir, un bouchon de blocage 10 est installé sur la tête 92. Ce bouchon comprend une partie annulaire 101 au centre de laquelle est défini un orifice 102 et à partir de laquelle s'étendent deux pions 103 et 104 qui sont diamétralement opposés par rapport à l'orifice 102. Les pions 103 et 104 s'étendent à partir de la même face de la partie 101. Ces pions sont séparés d'une distance d₁₀ sensiblement égale, ou légèrement supérieure, au diamètre D₉₂ de la tête 92 pris au niveau du fond des cannelures 921. Ainsi, les pions 103 et 104 peuvent être disposés de part et d'autre de la tête 92 en étant en partie engagés dans les cannelures 921, la forme des cannelures 921 étant choisie sensiblement complémentaire de celle des parties internes des pions 103 et 104 tournées vers l'orifice 102. En pratique, les cannelures 921 sont cylindriques et à génératrices en arc de cercle, alors que les pions 103 et 104 sont cylindriques à génératrice circulaire, avec un diamètre sensiblement égal à celui des cannelures. Le bouchon 10 et la tête 92 sont solidaires en rotation lorsque les pions 103 et 104 sont engagés dans les cannelures 921.

Par ailleurs, la portion 413 du perçage 41 est bordée par deux encoches 45 et 46 dans lesquelles sont engagés les pions 103 et 104 lorsque ceux-ci sont disposés autour de la tête 92 en place dans cette portion 413 qui forme un logement de réception de la tête 92. Les encoches 45 et 46 débouchent dans la portion 413. Ainsi, lorsque la tête 92 est reçue dans le logement formé par la portion 413, elle peut être coiffée du bouchon 10 avec les pions 103 et 104 partiellement engagés dans deux des cannelures 921 et, pour le reste, reçus dans les encoches 45 et 46.

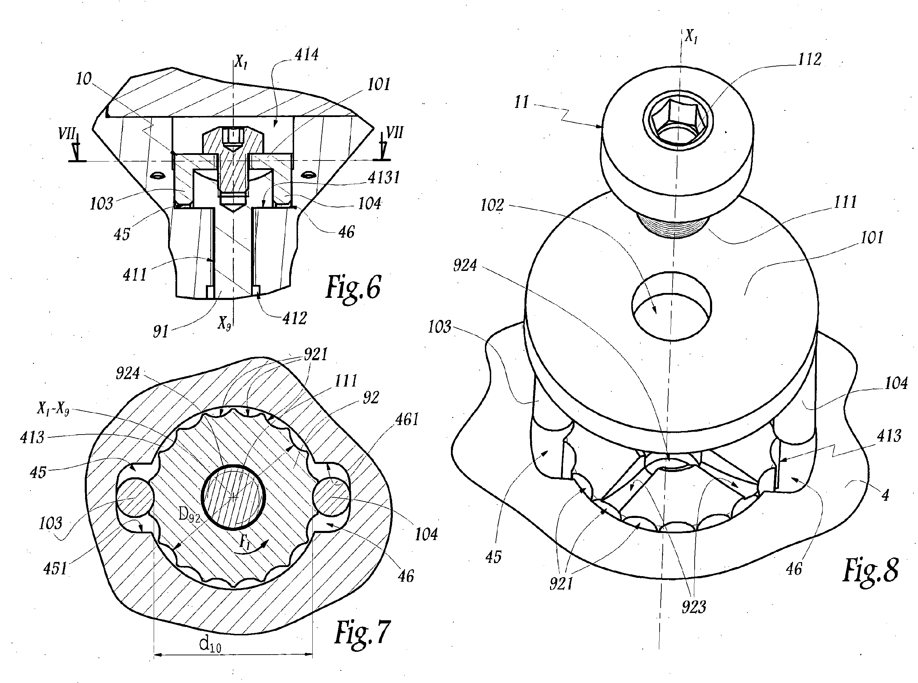

En cas d'amorce de dévissage de la vis 9, représentée par la flèche F₁ à la figure 7, les pions 103 et 104 sont entraînés en rotation par les cannelures autour de l'axe X₉ et viennent en appui sur des côtés 451 et 461 des encoches 45 et 46, ce qui a pour effet de bloquer en rotation la vis 9 qui ne peut alors pas être desserrée plus avant.

Le bouchon 10 constitue donc un premier moyen de blocage en rotation de la vis 9 par rapport à la partie métaphysaire 4 et, partant de là, des autres éléments du composant 1, à savoir la queue 2 et l'entretoise 3.

Une vis auxiliaire 11 est prévue pour immobiliser le bouchon 10 sur la tête 92. Pour ce faire, la tête 92 est pourvue d'un taraudage 924 aligné sur l'axe X₉ et dans lequel peut être engagée la tige 111 de la vis 11 en traversant l'orifice 102 du bouchon 10. Un fois le bouchon 10 en place sur la tête 92, avec ses pions engagés à la fois dans deux des cannelures 21 et dans les encoches 45 et 46, il peut ainsi être immobilisé sur la tête 92 en serrant la vis auxiliaire 11 dans le taraudage 924, au moyen d'une clef mâle insérée dans une empreinte polygonale 112 de la vis 11.

Ainsi, une fois le bouchon 10 et la vis auxiliaire 11 en place, la vis 9 ne risque pas de se desserrer avec un débattement angulaire supérieur à celui correspondant au déplacement des pions 103 et 104 pour venir au contact des côtés 451 et 461 des encoches 45 et 46. Ce débattement angulaire peut être relativement faible du fait d'un choix judicieux des dimensions respectives des pions 103 et 104 et des encoches 45 et 46.

En outre, le blocage en rotation de la vis 9 a lieu grâce à la coopération des pions 103 et 104, d'une part, et des encoches 45 et 46, d'autre part, même si la vis 9 n'est pas serrée à fond dans le taraudage 22. Ainsi, l'assemblage du composant 1 est pérenne, même si le chirurgien n'a pas complètement serré la vis dans le taraudage 22.

Selon une variante non représentée de l'invention, le bouchon 10 peut être maintenu en position sur et autour de la tête 92 par d'autres moyens que la vis auxiliaire 11, notamment par un talon prévu sur la surface arrière de l'insert 5, lequel talon vient alors en appui sur le bouchon 10 lorsque l'insert 5 est monté sur la partie 4.

L'invention a été représentée avec un composant 1 équipé d'une entretoise 3. Elle est toutefois applicable en l'absence d'une telle entretoise, la partie métaphysaire 4 étant alors directement en contact avec la queue 2.

Le bouchon 10 peut n'avoir qu'un seul pion de blocage du type des pions 103 et 104 ou, au contraire plus de deux pions, par exemple trois ou quatre pions qui sont alors avantageusement régulièrement répartis autour de l'axe central de l'orifice 102. La géométrie de la portion 413, notamment le nombre et la répartition des encoches 45, 46 et équivalentes, sont adaptées au nombre et à la répartition des pions.

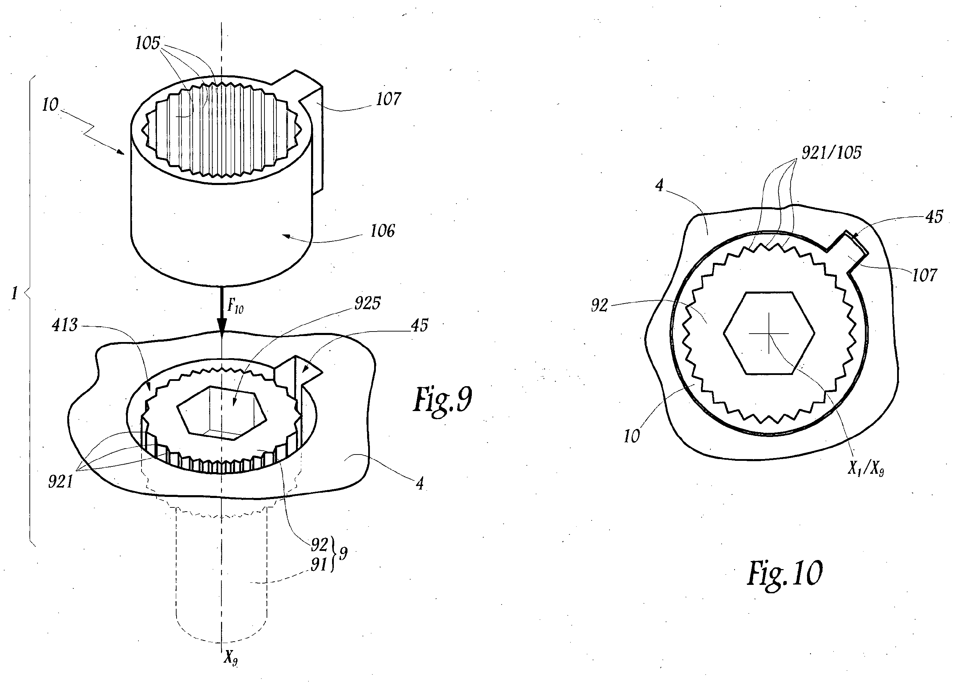

Dans les modes de réalisation représentés aux figures 9 à 13, les éléments analogues à ceux du premier mode de réalisation portent des références identiques.

Le composant 1 représenté partiellement aux figures 9 et 10 comprend une vis 9 dont la tige 91 est censée être vissée dans une queue d'ancrage non représentée. La tête 92 de la vis 9 est pourvue d'une denture périphérique externe 921 dont les dents ont une section globalement triangulaire. En configuration montée du composant 1, la tête 92 est reçue dans un logement 413 ménagé dans la partie métaphysaire 4 du composant 1. Le logement 413 est à section circulaire, avec un diamètre strictement supérieur à celui de la tête 92, alors qu'une encoche 45 est ménagée dans la partie 4 et débouche dans le logement 413.

Par ailleurs, une bague de blocage 10 est prévue pour être introduite dans le logement 413 autour de la tête 92, comme représenté par la flèche F₁₀ à la figure 9. La bague 10 est pourvue d'une denture interne 105 dont les dents sont à section triangulaire et de forme complémentaire des dents de la denture 921. Le rayon de la denture 105 est adapté au rayon de la tête 92, de telle sorte que, lorsque la bague est disposée autour de la tête 92, les dentures 921 et 105 sont en prise, ce qui induit une solidarisation en rotation de la tête 92 et de la bague 10 autour de l'axe X₉ de la vis 9.

La surface radiale externe 106 de la bague 10 est à section circulaire, avec un diamètre légèrement inférieur à celui du logement 413, à l'exception d'une partie où une nervure 107 fait saillie radialement par rapport à la surface 106 qui forme le bord extérieur de la bague 10. Lorsque la bague 10 est mise en place autour de la tête 92 dans le logement 413, la nervure 107 est engagée dans l'encoche 45. La nervure 107 s'étend radialement vers l'extérieur, par rapport à la surface 106, suffisamment pour bloquer en rotation la bague 10 par rapport à la partie 4 autour de l'axe longitudinal X₁ du composant 1 avec lequel est alors confondu l'axe X₉ de la vis 9.

Ainsi, une fois la vis 9 serrée par rapport à la queue d'ancrage et à la partie 4, sa rotation autour de l'axe X₉ est empêchée par la bague 10 dont la nervure 107 ne peut avoir qu'un faible débattement angulaire dans l'encoche 45.

Sur les figures 9 et 10, la référence 925 désigne une empreinte à six pans de manoeuvre de la vis 9 au moyen d'une clé Allen.

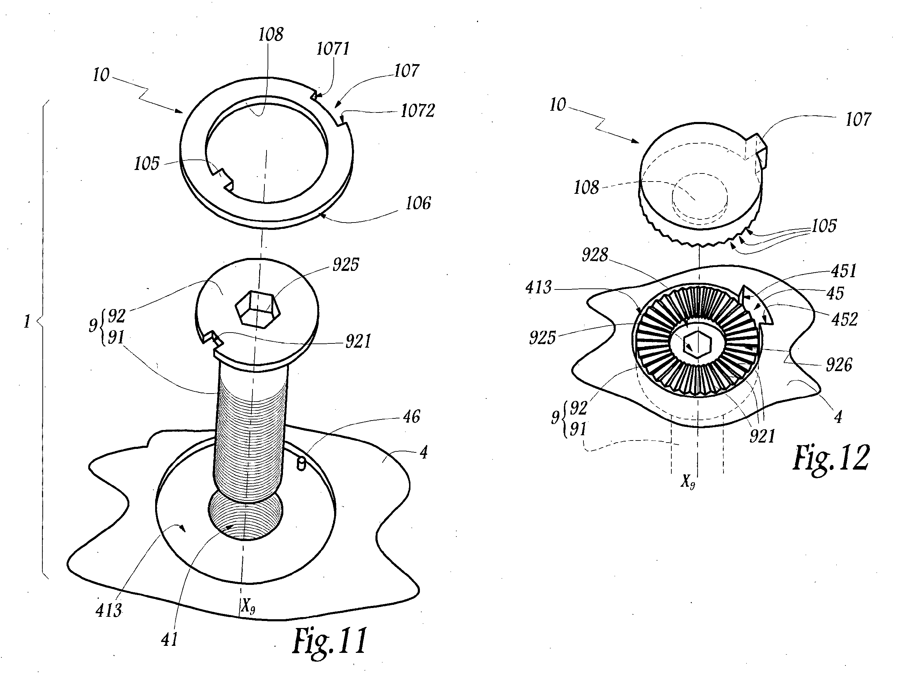

Dans le mode de réalisation de la figure 11, la vis 9 est pourvue d'une tige 91 et d'une tête 92 dont le bord périphérique externe est circulaire, à l'exception d'une encoche 921. La tête 92 est également équipée d'une empreinte 925 de manoeuvre à six pans.

La vis 9 est destinée à être insérée dans un perçage 41 dont la partie supérieure 413 forme un logement de réception de la tête 92. Dans ce logement est disposé un pion 46 qui s'étend parallèlement à un axe central X₁ du composant 1 et à un axe longitudinal X₉ de la vis 9 qui sont alors confondus.

Une bague de blocage 10 est prévue pour être disposée autour de la tête 92 dans le logement 413. Cette bague porte, sur son bord interne 108, une dent 105 dont les dimensions lui permettent d'être engagée dans l'encoche 921 avec un débattement angulaire nul ou minimum. Le bord périphérique externe 106 de la bague 10 est circulaire, à l'exception d'un secteur dans lequel est ménagé un dégagement 107 destiné à recevoir le pion 46 en configuration montée du composant 1. On note respectivement 1071 et 1072 les bords du dégagement 107. Lorsque la vis 9 est en place dans le perçage 41, avec sa tête 92 reçue dans le logement 413, il est possible d'engager la bague 10 autour de la tête 92 et dans le logement 413, en disposant la dent 105 dans l'encoche 921, alors que le dégagement 107 est placé autour du pion 46. Un éventuel mouvement de rotation de la vis 9 autour de l'axe X₉ est limité par l'appui de l'une des surfaces 1071 ou 1072 contre le pion 46.

Dans le mode de réalisation de la figure 12, la vis 9 du composant 1 comprend une tige 91 et une tête 92 pourvue d'une empreinte de manoeuvre 925. La tête 92 est équipée, sur sa face supérieure 926 opposée à la tige 91, d'une denture annulaire 921 ménagée autour de l'axe central X₉ de la vis et de l'empreinte 925.

Le logement 413 de réception de la tête 92, qui est ménagé dans la partie métaphysaire 4, est à section globalement circulaire avec une extension 45 qui communique avec la partie principale du logement 413.

Par ailleurs, un bouchon 10 est prévu pour être disposé au-dessus de la tête 92 en configuration assemblée du composant 1. Ce bouchon comprend une partie de centrage 108 destinée à être engagée dans un logement en creux de forme correspondante 928 formé sur la face 926. Par ailleurs, le bouchon 10 est pourvu d'une denture 105 dont la géométrie est adaptée à celle de la denture 921. Ainsi, lorsque la partie 108 est engagée dans le logement 928, les dentures 921 et 105 sont en prise et les pièces 9 et 10 sont solidarisées en rotation autour de l'axe X₉.

Le bord périphérique du bouchon 10 est équipé d'un taquet 107 qui est engagé dans l'encoche 45 en configuration montée du composant 1. La possibilité de rotation angulaire de la vis 9 autour de l'axe X₉ est ainsi limitée par l'appui du taquet 107 contre l'un ou l'autre des bords 451 et 452 de l'encoche 45.

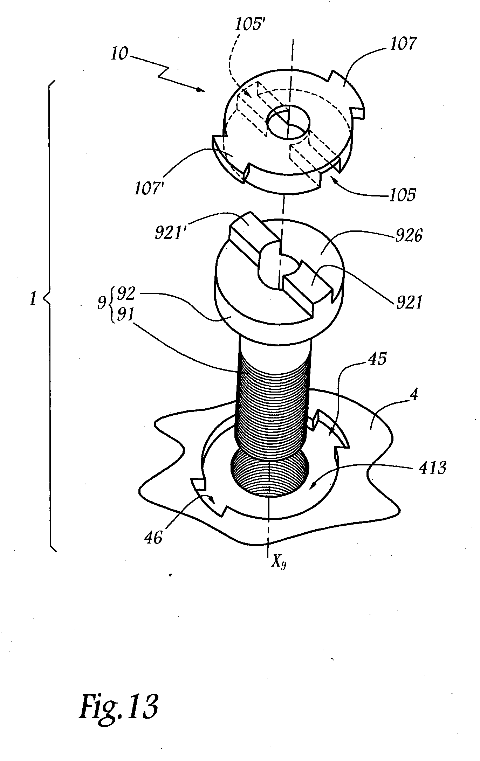

Dans le mode de réalisation de la figure 13, la vis 9 du composant 1 comprend une tige 91 et une tête 92 qui est pourvue de deux projections 921 et 921' qui s'étendent à partir de la face 926 de la tête 92 opposée à la tige 91.

Par ailleurs, un bouchon 10 est prévu pour être monté sur la tête 92 en configuration assemblée du composant 1. Ce bouchon comprend deux logements 105 et 105' dont la géométrie est adaptée pour recevoir les projections 921 et 921' lorsque le bouchon 10 est monté sur la tête 92. Ainsi, les pièces 9 et 10 sont solidarisées en rotation.

Par ailleurs, le bouchon 10 est équipé de deux ailettes 107 et 107' destinées à être engagées dans deux encoches 45 et 46 ménagées dans la partie métaphysaire 4, au voisinage d'un logement 413 de réception de la tête 92.

Comme précédemment, lorsque le bouchon 10 est monté sur la tête 92, sa rotation autour de l'axe X₉ est empêché par la coopération des ailettes 107 et 107', d'une part, et des encoches 45 et 46, d'autre part. Ceci permet de bloquer la rotation de la vis 9.

Dans les modes de réalisation représentés aux figures 9 à 13, il est possible d'utiliser un organe d'immobilisation de la bague ou du bouchon 10, similaire dans sa fonction à la vis auxiliaire 11 du premier mode de réalisation.

L'invention a été représentée dans le cas d'un composant huméral de prothèse d'épaule dont la surface articulaire est concave. Elle est toutefois applicable aux composants dont la surface articulaire est convexe. De façon générale, l'invention peut s'appliquer à tout composant de prothèse comprenant une queue d'ancrage, indépendamment de la géométrie de cette queue, et une partie métaphysaire fixées par une vis, quelle que soit la prothèse à laquelle il appartient. En particulier, l'invention s'applique également aux prothèses de coude, de hanche et de genou.

Selon une variante non représentée de l'invention, applicable à tous les modes de réalisation, la partie métaphysaire 4 peut former elle-même la surface articulaire 51.

## Revendications

1. Composant de prothèse articulaire comprenant une queue d'ancrage (2) apte à être ancrée dans un os, une partie métaphysaire (4), destinée à supporter un élément (5) formant une surface articulaire (51) ou formant elle-même une surface articulaire, une vis (9) de fixation de la partie métaphysaire sur la queue d'ancrage et des moyens (10, 11,) de blocage en rotation de la vis par rapport à la partie métaphysaire **caractérisé en ce que** les moyens (10, 11) de blocage en rotation comprennent une pièce de blocage (10) apte à venir en engagement, d'une part, avec au moins un relief correspondant (921) d'une tête (92) de la vis (9) et, d'autre part, avec au moins un relief correspondant (45, 46) de la partie métaphysaire (4).

2. Composant selon la revendication 1, **caractérisé en ce que** la pièce de blocage (10) est pourvue d'au moins un organe en saillie (103, 104) apte à être introduit entre une tête (92) de la vis (9) et la partie métaphysaire (4) au voisinage d'un logement (413) de réception de la tête de vis en configuration assemblée du composant (1).

3. Composant selon la revendication 2, **caractérisé en ce que** la pièce de blocage comprend au moins deux organes en saillie (103, 104) aptes à être introduits simultanément entre la tête de la vis (92) et la partie métaphysaire (4), de part et d'autre de cette tête.

4. Composant selon l'une des revendications 2 ou 3, **caractérisé en ce que** la surface radiale externe (922) de la tête de la vis (92) est pourvue de reliefs (921) aptes à coopérer avec le ou chaque organe en saillie (103, 104) pour solidariser en rotation la tête de la vis et la pièce de blocage (10).

5. Composant selon l'une des revendications 2 à 4, **caractérisé en ce que** la partie métaphysaire (4) comprend une ou des encoches (45, 46) en nombre égal au nombre d'organes en saillie (103, 104), **en ce que** la ou chaque encoche débouche dans le logement (413) de réception de la tête de la vis (92) et **en ce que** le ou chaque encoche est apte à recevoir partiellement le ou l'un des organes en saillie en configuration assemblée du composant (1).

6. Composant selon l'une des revendications 2 à 5, **caractérisé en ce que** les moyens de blocage comprennent des moyens (11, 924) de solidarisation de la pièce de blocage (10) et de la vis (9) dans une configuration où les moyens de blocage empêchent la rotation de la vis autour de son axe longitudinal (X₉).

7. Composant selon la revendication 6, **caractérisé en ce que** les moyens de solidarisation comprennent un taraudage (924) ménagé dans la tête (92) de la vis (9) et une vis auxiliaire (11) apte à être vissée dans ce taraudage en plaquant la pièce de blocage (10) contre la tête de la vis, dans une configuration où le ou les organes en saillie (103, 104) sont disposés entre la tête de la vis et la partie métaphysaire (4).

8. Composant selon la revendication 1, **caractérisé en ce que** la pièce de blocage (10) est pourvue de :
- au moins un premier relief (105) apte à coopérer avec au moins un relief correspondant (921) de la tête (92) de la vis (9) pour solidariser en rotation la pièce de blocage et la vis et
- au moins un second relief (107) apte à coopérer avec au moins un relief (45, 46) fixe par rapport à la partie métaphysaire (4) pour bloquer la rotation de la pièce de blocage.

9. Composant selon la revendication 8, **caractérisé en ce que** la pièce de blocage est une bague (10), **en ce que** son premier relief (105) est interne et **en ce que** le relief correspondant est un relief radial externe (921) de la tête de la vis.

10. Composant selon la revendication 9, **caractérisé en ce que** la bague (10) est équipée d'une denture périphérique interne (105), qui forme plusieurs premiers reliefs, et **en ce que** la tête de la vis est équipée d'une denture périphérique externe (921), qui forme plusieurs reliefs correspondant aux premiers reliefs.

11. Composant selon la revendication 9, **caractérisé en ce que** le premier relief de la bague est une dent (105), alors que le relief correspondant de la tête (92) de la vis (9) est une encoche (921).

12. Composant selon la revendication 8, **caractérisé en ce que** la pièce de blocage est un bouchon (10) qui recouvre la tête (92) de la vis (9), **en ce que** le premier relief (105) est ménagé sur un côté du bouchon tourné vers la tête (92) de la vis et **en ce que** le relief correspondant (921) est ménagé sur une face (926) de la tête de la vis opposée à sa tige (91).

13. Composant selon l'une des revendications 8 à 12, **caractérisé en ce que** le deuxième relief (107) de la pièce de blocage (10) s'étend sur le, ou à partir du, bord périphérique externe (106) de cette pièce.

14. Composant selon l'une des revendications précédentes, **caractérisé en ce qu'**il comprend une entretoise (3) destinée à être disposée entre la queue d'ancrage (2) et la partie métaphysaire (4), cette entretoise étant pourvue, sur ses faces opposées (33, 34) tournées respectivement vers la queue d'ancrage et vers la partie métaphysaire, de reliefs (331, 341) destinés à venir en prise avec des reliefs correspondants (231, 421), ménagés respectivement sur la queue d'ancrage et sur la partie métaphysaire, en empêchant une rotation de la partie métaphysaire par rapport à la queue d'ancrage, autour d'un axe longitudinal (X₃) de l'entretoise.

15. Prothèse articulaire, notamment prothèse d'épaule, comprenant un composant (1) selon l'une des revendications précédentes.

## Patentansprüche

1. Komponente für eine Gelenkprothese, ein Verankerungsende (2), das geeignet ist, in einem Knochen verankert zu werden, ein metaphysäres Teil (4), das vorgesehen ist, ein eine Gelenkfläche (51) bildendes Element (5) zu lagern oder das selbst eine Gelenkfläche bildet, eine Schraube (9) zum Befestigen des metaphysären Teils an dem Verankerungsende und Mittel (10, 11) zum Blockieren der Schraube hinsichtlich einer Rotation in Bezug auf das metaphysäre Teil umfassend, **dadurch gekennzeichnet, dass** die Mittel (10, 11) zum Blockieren hinsichtlich der Rotation ein Blockierstück (10) umfassen, das geeignet ist, einerseits in Eingriff mit mindestens einem korrespondierenden Relief (921) eines Kopfes (92) der Schraube (9) und andererseits mit mindestens einem korrespondierenden Relief (45, 46) des metaphysären Teils (4) zu kommen.

2. Komponente nach Anspruch 1, **dadurch gekennzeichnet, dass** das Blockierstück (10) mit mindestens einem vorspringenden Element (103, 104) versehen ist, das geeignet ist, zwischen einen Kopf (92) der Schraube (9) und das metaphysäre Teil (4) benachbart zu einem Raum (413) zur Aufnahme des Kopfes der Schraube im zusammengesetzten Zustand der Komponente (1) eingeführt zu werden.

3. Komponente nach Anspruch 2, **dadurch gekennzeichnet, dass** das Blockierstück mindestens zwei vorspringende Elemente (103, 104) umfasst, die geeignet sind, gleichzeitig zwischen den Kopf der Schraube (92) und das metaphysäre Teil (4) beidseitig dieses Kopfes eingeführt zu werden.

4. Komponente nach einem der Ansprüche 2 oder 3, **dadurch gekennzeichnet, dass** die radiale äußere Fläche (922) des Kopfes der Schraube (92) mit Reliefs (921) versehen ist, die geeignet sind, mit dem oder jedem vorspringenden Element (103, 104) zusammenzuarbeiten, um den Kopf der Schraube und das Blockierstück (10) hinsichtlich der Rotation zu verbinden.

5. Komponente nach einem der Ansprüche 2 bis 4, **dadurch gekennzeichnet, dass** das metaphysäre Teil (4) eine oder mehrere Ausbuchtungen (45, 46) mit einer Anzahl gleich der Anzahl der vorspringenden Elemente (103, 104) umfasst, dass die oder jede Ausbuchtung in den Raum (413) zur Aufnahme des Kopfes der Schraube (92) mündet und dass die oder jede Ausbuchtung geeignet ist, teilweise das oder eines der vorspringenden Elemente im zusammengesetzten Zustand der Komponente (1) aufzunehmen.

6. Komponente nach einem der Ansprüche 2 bis 5, **dadurch gekennzeichnet, dass** die Mittel zum Blockieren Mittel (11, 924) zum Befestigen des Blockierstücks (10) und der Schraube (9) in einer Konfiguration umfassen, in der die Mittel zum Blockieren die Drehung der Schraube um ihre Längsachse (X₉) verhindert.

7. Komponente nach Anspruch 6, **dadurch gekennzeichnet, dass** die Mittel zum Befestigen ein Gewinde (924), das in dem Kopf (92) der Schraube (9) eingearbeitet ist, und eine Hilfsschraube (11) umfassen, die geeignet ist, in dieses Gewinde eingeschraubt zu werden, wobei das Blockierstück (10) gegen den Kopf der Schraube in einer Konfiguration gedrückt wird, in der das oder die vorspringenden Elemente (103, 104) zwischen dem Kopf der Schraube und dem metaphysären Teil (4) angeordnet sind.

8. Komponente nach Anspruch 1, **dadurch gekennzeichnet, dass** das Blockierstück (10) versehen ist mit:
- mindestens einem ersten Relief (105), das geeignet ist, mit mindestens einem korrespondierenden Relief (921) des Kopfes (92) der Schraube (9) zusammenzuarbeiten, um das Blockierstück und die Schraube drehfest zu verbinden, und
- mindestens ein zweites Relief (107), das geeignet ist, mit mindestens einem in Bezug auf das metaphysäre Teil (4) feststehenden Relief (45, 46) zusammenzuarbeiten, um die Drehung des Blockierstücks zu blockieren.

9. Komponente nach Anspruch 8, **dadurch gekennzeichnet, dass** das Blockierstück ein Ring (10) ist, dass sein erstes Relief (105) innenliegend ist und dass das korrespondierende Relief ein äußeres radiales Relief (921) des Kopfes der Schraube ist.

10. Komponente nach Anspruch 9, **dadurch gekennzeichnet, dass** der Ring (10) mit einer inneren Umfangsverzahnung (105) ausgerüstet ist, die mehrere erste Reliefs bildet, und dass der Kopf der Schraube mit einer äußeren Umfangsverzahnung (921) ausgerüstet ist, die mehrere zu den ersten Reliefs korrespondierende Reliefs bildet.

11. Komponente nach Anspruch 9, **dadurch gekennzeichnet, dass** das erste Relief des Rings ein Zahn (105) ist, während das korrespondierende Relief des Kopfes (92) der Schraube (9) eine Einbuchtung (921) ist.

12. Komponente nach Anspruch 8, **dadurch gekennzeichnet, dass** das Blockierstück ein Stopfen (10) ist, der den Kopf (92) der Schraube (9) bedeckt, dass das erste Relief (105) an einer Seite des Stopfens, die zu dem Kopf (92) der Schraube gerichtet ist, eingearbeitet ist und dass das korrespondierende Relief (921) an einer Fläche (926) des Kopfes der Schraube entgegengesetzt zu ihrem Schaft (91) eingearbeitet ist.

13. Komponente nach einem der Ansprüche 8 bis 12, **dadurch gekennzeichnet, dass** das zweite Relief (107) des Blockierstücks (10) sich an dem oder von dem äußeren Umfangsrand (106) dieses Stücks erstreckt.

14. Komponente nach einem der vorhergehenden Ansprüche, **dadurch gekennzeichnet, dass** sie ein Distanzstück (3) umfasst, das vorgesehen ist, zwischen dem Verankerungsende (2) und dem metaphysären Teil (4) angeordnet zu werden, wobei dieses Distanzstück auf seinen entgegengesetzten Flächen (33, 34), die jeweils zu dem Verankerungsende und zu dem metaphysären Teil gerichtet sind, mit Reliefs (331, 341) versehen ist, die vorgesehen sind, mit korrespondierenden Reliefs (231, 421) in Eingriff zu kommen, die jeweils an dem Verankerungsende und dem metaphysären Teil eingearbeitet sind, wobei sie eine Drehung des metaphysären Teils in Bezug auf das Verankerungsende um eine Längsachse (X₃) des Distanzstücks verhindern.

15. Gelenkprothese, insbesondere Schulterprothese, eine Komponente (1) gemäß einem der vorhergehenden Ansprüche umfassend.

## Claims

1. Joint prosthesis component, comprising an anchoring stem (2) arranged to be anchored in a bone, a metaphyseal part (4) which is provided for supporting an element (5) forming a joint surface (51) or which itself forms a joint surface, a screw (9) for fixing the metaphyseal part onto the anchoring stem, and means (10, 11) for blocking rotation of the screw relative to the metaphyseal part, **characterised in that** the rotation-blocking means (10, 11) comprise a blocking part (10) arranged to come into engagement with, on the one hand, at least one corresponding relief (921) of a head (92) of the screw (9) and with, on the other hand, at least one corresponding relief (45, 46) of the metaphyseal part (4).

2. Component according to claim 1, **characterised in that** the blocking part (10) is provided with at least one projecting member (103, 104) arranged to be introduced between a head (92) of the screw (9) and the metaphyseal part (4) in the vicinity of a seating (413) for receiving the screw head in the assembled configuration of the component (1).

3. Component according to claim 2, **characterised in that** the blocking part comprises at least two projecting members (103, 104) arranged to be introduced simultaneously between the screw head (92) and the metaphyseal part (4), on one and the other side of that head.

4. Component according to one of claims 2 or 3, **characterised in that** the external radial surface (922) of the screw head (92) is provided with reliefs (921) arranged to co-operate with the or each projecting member (103, 104) in order to lock together the screw head and the blocking part (10) in a manner preventing relative rotation.

5. Component according to one of claims 2 to 4, **characterised in that** the metaphyseal part (4) comprises one or more indentation(s) (45, 46), the number thereof being equal to the number of projecting members (103, 104); **in that** the or each indentation is open to the seating (413) for receiving the screw head (92); and **in that** the or each indentation is arranged to partially receive the or one of the projecting member(s) in the assembled configuration of the component (1).

6. Component according to one of claims 2 to 5, **characterised in that** the blocking means comprise means (11, 924) for locking together the blocking part (10) and the screw (9) in a configuration wherein the blocking means prevent rotation of the screw about its longitudinal axis (X₉).

7. Component according to claim 6, **characterised in that** the locking means comprise an internal thread (924) provided in the head (92) of the screw (9) and an auxiliary screw (11) arranged to be screwed into that internal thread, thereby pressing the blocking part (10) against the screw head, in a configuration wherein the projecting member(s) (103, 104) is/are arranged between the screw head and the metaphyseal part (4).

8. Component according to claim 1, **characterised in that** the blocking part (10) is provided with:
- at least one first relief (105) arranged to co-operate with at least one corresponding relief (921) of the head (92) of the screw (9) in order to lock together the blocking part and the screw in a manner preventing relative rotation and
- at least one second relief (107) arranged to co-operate with at least one relief (45, 46) which is fixed relative to the metaphyseal part (4) in order to block rotation of the blocking part.

9. Component according to claim 8, **characterised in that** the blocking part is a ring (10); **in that** its first relief (105) is internal; and **in that** the corresponding relief is an external radial relief (921) of the screw head.

10. Component according to claim 9, **characterised in that** the ring (10) is provided with an internal peripheral toothed arrangement (105) which forms a plurality of first reliefs; and **in that** the screw head is provided with an external peripheral toothed arrangement (921) which forms a plurality of reliefs corresponding to the first reliefs.

11. Component according to claim 9, **characterised in that** the first relief of the ring is a tooth (105) whilst the corresponding relief of the head (92) of the screw (9) is an indentation (921).

12. Component according to claim 8, **characterised in that** the blocking part is a stopper (10) which overlies the head (92) of the screw (9); **in that** the first relief (105) is provided on a side of the stoopper which faces the screw head (92); and **in that** the corresponding relief (921) is provided on a face (926) of the screw head which is located opposite its shank (91).

13. Component according to one of claims 8 to 12, **characterised in that** the second relief (107) of the blocking part (10) extends on or starting from the external peripheral edge (106) of that part.

14. Component according to one of the preceding claims, **characterised in that** it comprises a spacer sleeve (3) provided for placing between the anchoring stem (2) and the metaphyseal part (4), that spacer sleeve being provided, on its opposite faces (33, 34) respectively facing the anchoring stem and the metaphyseal part, with reliefs (331, 341) arranged to come into engagement with corresponding reliefs (231, 421) provided respectively on the anchoring stem and on the metaphyseal part, thereby preventing rotation of the metaphyseal part relative to the anchoring stem about the longitudinal axis (X₃) of the spacer sleeve.

15. Joint prosthesis, especially a shoulder prosthesis, comprising a component (1) according to one of the preceding claims.
